# EUROPEAN PATENT APPLICATION

(11) **EP 0 794 422 A2**
(43) Date of publication of application: **10.09.1997**
(21) Application number: 97200641.5
(22) Date of filing: 04.03.1997
(51) Int. Cl.: G01N 1/28, G01N 1/32, B25J 5/00, G01N 33/20

(54) **Robot and method for final product control**

(30) Priority: 06.03.1996 SE 9600858
(71) Applicant: Avesta Sheffield Aktiebolag, S-103 27 Stockholm (SE)
(72) Inventor: Andersson, Stig, 774 40 Avesta (SE); Johansson, Lennarth, 775 70 Krylbo (SE); Karlsson, Johan, 774 41 Avesta (SE)
(74) Representative: Hynell, Magnus

(57) **Abstract**

A robot for spectrometric analysis of metal samples has a fixed frame and a movable frame. The fixed frame 16 contains a holder 25 for a sheet sample and a brushing machine 30. The movable frame 17 contains a carriage 36 for the spark probe 13 of the spectrometer 12 and a grinding machine 40 which are both movable on guiders 35, 39 which are perpendicular to the guiders 16 on the fixed frame along which the movable frame 17 moves. In a first relative position between the frames, the working surface of the probe is brushed clean and the sheet sample is ground clean. In a second relative position between the frames the probe moves up against the sheet sample for measurement. The sheet sample is secured into position and is not released until the spectrometer has indicated an accepted sample.

## Description

### FIELD OF INVENTION

The present invention relates to a robot for spectrometric analysis of metal samples and to a method for carrying out final control of metal products.

### DESCRIPTION OF THE BACKGROUND ART

In order to reduce the risk for mix-ups it is necessary to check that one has the correct steel grade both on delivery from steel plant to customer and also in production e.g. between the hot rolling mill and cold rolling mill. This can be done suitably as an analysis check with a spectrometer and this is usually done completely manually. Analyses are also made in other cases with the spectrometer.

### DESCRIPTION OF THE INVENTION

The objective of the present invention is to simplify analysis measurement of metal samples, to make it more reliable and to make a mix-up check simple and reliable.

### BRIEF DESCRIPTION OF THE DRAWINGS

A preferred embodiment of the invention will now be described with reference to the accompanying drawings, in which
- Figure 1: shows a schematic and fragmentary sketch in side view of an example of a robot with attached spectrometer according to the invention; and
- Figure 2: shows a flow chart of a measurement cycle which can be performed by the robot.

### DESCRIPTION OF A PREFERRED EMBODIMENT

The spectrometer shown in the drawing is identified generally by the reference numeral 12, with an assay probe 13. The spectrometer can be of type SPECTROTEST Jr which is an optical emission spectrometer (OES). The assay probe melts by sparking a small amount of metal in the sample, and the light is transferred through an optical cable 14 to the spectrometer 12 where the intensities for the spectra of different metals are analysed and translated to metal contents. It is possible to program the analysis limits of the different steel grades so that a signal indicating an accepted or unaccepted measurement is obtained.

The robot has a fixed frame of which only a pair of guides 16 is shown. A movable frame 17 is mounted on the guiders 16. Only the bottom plate 18 of the movable frame 17 is shown with sliding components 19, 20 on the guiders 16 and a back wall 21. A compressed-air cylinder 22 is connected to slide the movable frame 17 along the guiders 16 of the fixed frame.

A sample holder 25 is mounted onto the fixed frame. It has a compressed-air cylinder 26 with a plate 27 with which it holds the sheet-metal sample 28 in place. A brushing machine 30 is also on the fixed frame, it has an axially rotating brush 31 and a compressed-air cylinder 32 the piston rod of which carries a plate 33.

The movable frame 17 has a guider 35 on which a carriage 36 for the assay probe 13 is mounted. A compressed-air cylinder 37 is connected to advance the probe carriage 36 along the guider 35. A grinding machine 40 is mounted on a guider 39 which is also supported by the movable frame 17. It can be advanced along the guider 39 by a compressed-air cylinder 42. The grinding machine 40 has a rotating grinding wheel 41 with loose radial grinding belts which give a relatively large grinding surface. The guiders 35 and 39 are in parallel with each other but perpendicular to the guiders 16 of the fixed frame. Two compressed-air cylinders are mounted onto the movable frame 17 parallel to each other so that only one of them 45 can be seen in the figure. It has a stop lug 46 which, when the piston rod is projected as in the figure, is in position to stop against a cross bar 47 attached between the guider pair 16 of the fixed frame. The lug 48 on the other cylinder can be seen in the figure where it is shown in non-operative position. The lugs 46, 48 are different sizes so that they will define two different stop positions for the movement to the right of the movable frame 17 in the figure. When both lugs 46, 48 are in their non-operative positions, the bottom plate 18 will rest against the cross bar 47 which gives a third stop position.

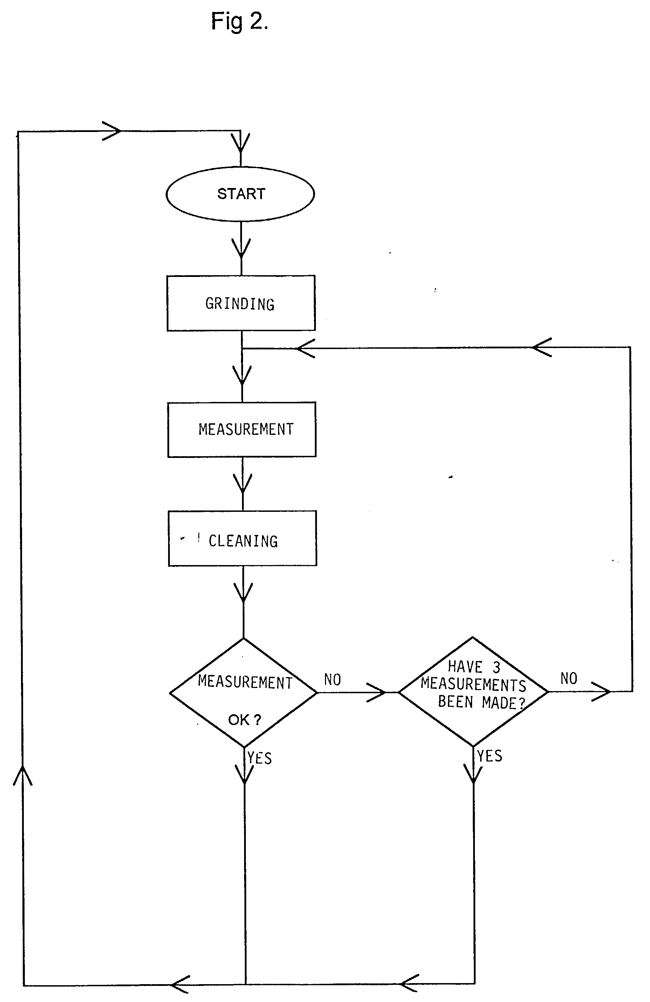

Figure 2 is a flow chart showing the sequences in a measurement operation and such a measurement operation shall now be described. The steering system for sequential control is of a conventional type and is therefore not shown.

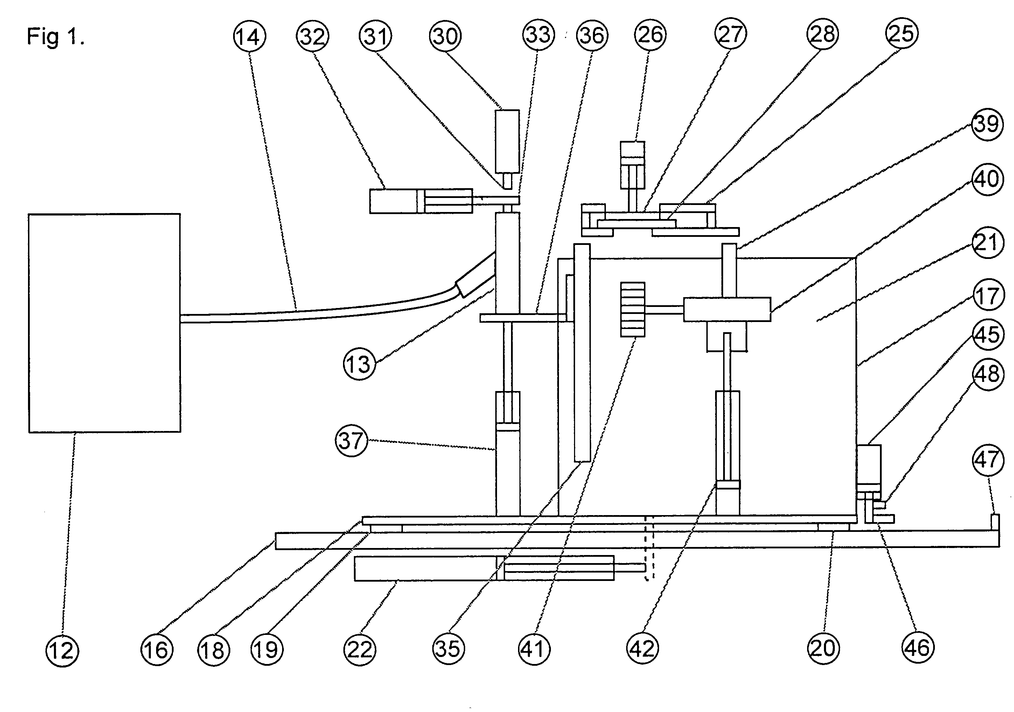

In Figure 1 the robot is shown in its idle position, in which the probe 13 is in line with the brushing machine 30 and the grinding wheel 41 is directly in front of the sample holder 25. The probe 13 is moved towards the plate 33 so that the working surface of the probe is protected and the grinding machine 40 is in a retired idle position.

A measurement sequence begins when the operator puts a sheet-metal sample 28 into the sample holder 25 and adjusts the spectrometer for the correct steel grade. When he then pushes the start button, the cylinder 26 clamps the sheet sample 28 and the grinding machine 40 starts and is brought upwards by the cylinder 42 so that the grinding wheel 41 grinds smooth an approximately square area on the sheet sample 28. Thereafter the grinding machine 40 goes down again as the probe carriage 36 also goes down. The cylinder 22 then advances the movable frame 17 to the right until the stop lug 46 of the cylinder 45 stops against the cross bar 47. In this relative position between the fixed frame 16 and the movable frame 17, the probe 13 is in front of the sheet sample 28 and is brought up against the sheet sample and begins sparking. If the measured analysis falls within the programmed analysis limits, it indicates that no mix-up has occurred and that the material is correct. The spectrometer 12 gives a clear signal and initiates release of the sheet sample 28 and the return of the movable frame 17 to the initial position shown in the figure. The cylinder 32 has withdrawn its plate 33 and the probe 13 is brought up against the brushing machine which is started and brushes clean the top surface of the probe so that it is clean before the next measurement and does not itself add contamination. The cylinder 32 brings the plate 33 forth when the probe 13 has gone down and thereafter the probe goes up again to bear on the plate 33 where the top surface of the probe is protected. Shrouding gas e.g. argon is introduced through a hose which lies within the same casing as the optical cable, in a low continuous flow towards the top surface of the probe and protects it from atmospheric oxygen.

If the sample is unaccepted then the probe does not go up into the idle position but the movable frame 17 is advanced to the right in the figure to a second measurement position. The cylinder 45 first pulls up the stop lug 46 and the next cylinder brings down another stop lug 48 so that the frame 17 comes to an operative position somewhat more than 1 cm to the side of the previous operative position. In this way the probe 13 can make a new measurement on a clean surface right beside the area where it took the first measurement.

If this measurement is also unaccepted, the robot repeats the measurement one more time; this time with both stop lugs 46, 48 in non-operative positions. If the third measurement is also unaccepted, the robot stops in idle position with the sheet sample 28 in grip. The operator must therefore give an extra command so that the sheet sample can be released, and he can therefore not avoid noticing that the sample is not accepted, which increases the reliability.

In the above example both the brushing machine 30 and sample holder 25 are fixed while the probe carriage 36 and grinding machine 40 are movable. One can of course arrange for the opposite so that the brushing machine 30 and sample holder 25 become movable. The different power units have been described as compressed-air cylinders, but they can be other types of power units.

It will be understood that the invention is not restricted to the described and illustrated embodiment and that modifications can be made within the scope of the following claims.

## Claims

1. Robot for spectrometric analysis of metal samples, **characterized** in that it comprises a first frame (16) which contains a motor-driven brush (31) and a sample holder (25), and a second frame (17) which contains an assay probe carriage (36) for an assay probe (13), which is connected to a spectrometer (12), and a grinding machine (40) at which either the brush or probe carriage and either the sample holder or grinding machine can be moved in parallel movement with each other in a first direction and the frames (16,17) are shiftable relative to each other in a direction perpendicular to the first-mentioned direction between a first position, in which the probe carriage (36) and the brush (31) can cooperate so that the brush can brush clean the working surface of the probe and the grinding machine (40) and the probe carriage (25) can cooperate so that the grinding machine can grind clean a sample (28) secured in the sample holder, and a second position in which the probe carriage and sample holder can cooperate so that the probe comes into contact with the sample.

2. Robot according to claim 1, **characterized** in that the sample holder (25) has a device (26, 27) for securing of the sample (28) in the sample holder and the control device of the robot is arranged to control the sample holder to release the sample when the spectrometer (12) signals an approved sample.

3. Robot according to claims 1 or 2, **characterized** in that the frames (16, 17) have two or more fixed positions adjacent to each other so that the probe (13) has alternative points of contact with the sample (28).

4. Robot according to claim 3, **characterized** in that it has a control device arranged to sequentially control the robot to initiate a measurement with the frames (16, 17) in a first fixed relative position and to move the frames to a second fixed relative position and to initiate a new measurement if the first measurement fails to result in an accepted analysis.

5. Robot according to claim 4, **characterized** in that the controlling device is arranged to sequentially control the robot to move the frames (16, 17) to a third fixed relative position and to initiate a new measurement if the second measurement fails to result in an accepted analysis.

6. Robot for spectrometric analysis of metal samples, **characterized** in that it includes a first frame (6) which contains a motor-driven brush (31) and a sample holder (25), a second frame (17) which contains an assay probe carriage (36) and a grinding machine (40) which are each controlled in parallel movement with each other in a first direction, the frames (16, 17) are shiftable relative to each other in a direction perpendicular to the first-mentioned direction between a first position in which a probe carriage (36) can be advanced in the first-mentioned direction towards and away from a position in which its probe (13) is in contact with the brush (31) and the grinding machine (40) can be advanced in the first-mentioned direction towards and away from a position in which its grinding means (41) is in contact with a sample (28) secured in a sample holder (25) and a second position in which the probe carriage (36) can be advanced in the first-mentioned direction and to bring the probe (13) into and out of contact with the sample.

7. Method for final product control of metallic products by analysis testing with spectrometer, **characterized** in that accepted analysis limits are programmed into the spectrometer (12) and a sample holder (25) is used to secure the sample (28) and is connected to the spectrometer so that it releases the sample when the spectrometer signals an accepted sample.
